# EUROPÄISCHE PATENTSCHRIFT

(11) **EP 0 883 619 B1**
(45) Veröffentlichungstag und Bekanntmachung des Hinweises auf die Patenterteilung: **16.01.2002**
(21) Anmeldenummer: 97903288.5
(22) Anmeldetag: 15.02.1997
(51) Int. Cl.: C07D 489/04, A61K 31/485

(54) **MORPHIN- UND DIAMORPHINSALZE ANIONISCHER NICHT-NARKOTISCHER ANALGETIKA VOM TYP DER SUBSTITUIERTEN CARBONSÄUREN**
MORPHINE AND DIAMORPHINE SALTS OF ANIONIC NON-NARCOTIC ANALGESICS OF THE SUBSTITUTED CARBOXYLIC ACID TYPE
SELS DE MORPHINE ET DE DIAMORPHINE D'ANALGESIQUES ANIONIQUES NON NARCOTIQUES DU TYPE DES ACIDES CARBOXYLIQUES SUBSTITUES

(30) Priorität: 28.02.1996 DE 19607395
(43) Veröffentlichungstag der Anmeldung: 16.12.1998
(73) Patentinhaber: LTS Lohmann Therapie-Systeme AG, 56626 Andernach (DE)
(72) Erfinder: ASMUSSEN, Bodo, D-56170 Bendorf (DE); MÜLLER, Walter, D-56564 Neuwied (DE); RIESS, Walter, D-91522 Ansbach (DE)
(74) Vertreter: Flaccus, Rolf-Dieter, Dr.
(86) Internationale Anmeldenummer: EP9700711
(87) Internationale Veröffentlichungsnummer: WO9731918

(56) Entgegenhaltungen:
- EP-A- 0 137 600
- EP-A- 0 472 501
- EP-A- 0 649 657
- WO-A-92/15332
- WO-A-95/04058
- US-A- 4 404 209
- DATABASE WPI Section Ch, Week 9114 Derwent Publications Ltd., London, GB; Class B05, AN 91-102327 XP002031781 & ZA 8 903 422 A (ADCOCK-INGRAM PHARM) , 30.Januar 1991

## Beschreibung

Gegenstand der vorliegenden Erfindung sind Morphin- und Diamorphinsalze anionischer nicht-narkotischer Analgetika vom Typ der substituierten Carbonsäuren, vorzugsweise die Morphin- und Diamorphinsalze des Diclofenac (Formel 1), Verfahren zu deren Herstellung, diese Salze zur Behandlung von Krankheiten, sowie pharmazeutische Präparate, die diese Salze enthalten.

Die EP 0472 501 A beschreibt das Codeinsalz der [2[(2,6-Dichlorphenyl)-amino]-phenyl]-essigsäure (Diclofenac), die WO 95 04 058 A beschreibt das Codeinsalz der 2-(3-Benzoylphenyl)-propionsäure (Ketoprofen).

Eines der häufigsten Symptome einer Krankheit oder einer Schädigung ist der Schmerz . Auch wenn der Schmerz als Warn- und Schutzfunktion des Organismus zu verstehen ist, verlangt der betroffene Patient in der Regel nach schmerz-stillenden, zumindest aber schmerzlindernden Substanzen. Ein für die Medizin zentrales Anliegen besteht daher darin, solche Substanzen bereitzustellen. Die Aufgabe dieser Substanzen, sogenannte Analgetika, besteht darin, in therapeutischen Dosen die Schmerzempfindung zu verringern bzw. zu unterdrücken, ohne bei einer solchen Dosierung allgemeinnarkotisch zu wirken. Aufgrund der Wirkungsstärke, des Wirkungsmechanismus und der Nebenwirkungen werden zwei Gruppen von Analgetika unterschieden: zentral angreifende, stark wirkende und vorwiegend peripher angreifende, schwach bis mittelstark wirkende Analgetika. Vielfach treten beim Einsatz zentral angreifender Wirkstoffe Gewöhnungserscheinungen auf, die sich bis zur Abhängigkeit steigern können. Ein Beispiel für einen zentral angreifenden Wirkstoff mit eben diesem Risiko ist das Morphin (Formel 3a). Morphin ist in Form seiner anorganischen Salze, beispielsweise seines Hydrochlorids oder Sulfats, zur Beherrschung starker posttraumatischer oder post-operativer Schmerzen sowie chronischer Schmerzen, beispielsweise im Zustand fortgeschrittener Krebsleiden, für parenterale und perorale Anwendung im Handel. Ein Derivat des Morphins, das Diacetylmorphin (Formel 3b), das auch unter den Namen Diamorphin oder Heroin bekannt ist, wird ohne jegliche pharmakologische, pharmazeutische und pharmakokinetische Kontrolle in der Drogenszene gehandelt und konsumiert. Seine qualifizierte Verwendung in der Therapie der Drogenabhängigkeit ist ein noch nicht gelöstes wissenschaftliches und soziologisches Problem.

Zur Minderung des Risikos der Morphinabhängigkeit im klinischen Gebrauch von Morphinpräparaten werden gleichzeitig oder abwechselnd andere Analgetika, vorzugsweise nicht-narkotische, peripher wirksame Präparate, verabreicht. Die große Zahl der peripher wirksamen Analgetika, ihre unterschiedliche Potenz und infolgedessen unterschiedliche Dosierung führen zu großer Unsicherheit in der Wahl der zu kombinierenden Präparate und verursachen Belastungen des Patienten durch die Menge der einzunehmenden Arzneien.

Aufgabe der vorliegenden Erfindung ist die.Bereitstellung neuer Analgetika, die bei maximalem analgetischem Effekt minimale Nebenwirkungen aufweisen.

Gelöst wird diese Aufgabe durch die in den Ansprüchen charakterisierten neuen Wirkstoffe und durch die pharmazeutischen Präparate, die diese Wirkstoffe enthalten.

Die erfindungsgemäßen Wirkstoffe sind die Morphin- und Diamorphinsalze anionischer nicht-narkotischer Analgetika vom Typ der substituierten Carbonsäuren. Als anionische nicht-narkotische Analgetika vom Typ der substituierten Carbonsäuren können eingesetzt werden: Diclofenac, Indometacin, Sulindac, Ketoprofen oder Fenbufen.

Vorzugsweise umfaßt die Erfindung die Salze Morphin-Diclofenat (Formel 1a) und Diamorphin-Diclofenat (Formel 1b), bestehend aus dem anionischen peripher wirksamen Analgetikum Diclofenac ([2-[(2,6-Dichlorphenyl)-amino]-phenyl]-essigsäure (Formel 2)) und dem kationischen, zentral wirksamen Analgetikum Morphin (Formel 3a) bzw. seinem Derivat Diacetylmorphin (Formel 3b), auch Diamorphin genannt. In der Formel 1 kann R = H oder CH₃CO bedeuten. Steht R für H, handelt es sich um das Morphin-Diclofenat (Formel 1a); steht R für CH₃CO, handelt es sich um das Diamorphin-Diclofenat (Formel 1b).

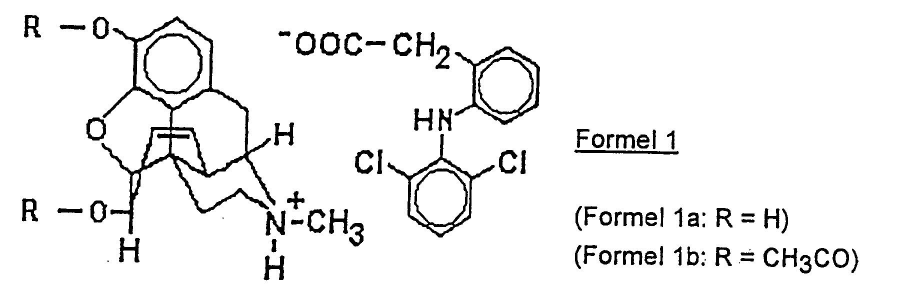

Die obengenannten nicht-narkotischen Analgetika vom Typ der substituierten Carbonsäuren sowie deren Salze sind bekannt. Die Natrium-, Kalium- und Diethylammoniumsalze des Diclofenac (Formel 2) ([2-[(2,6-Dichlorphenyl)-amino]-phenyl]-essigsäure) werden beispielsweise zur Behandlung schmerzhafter entzündlicher Prozesse eingesetzt. Präparate zur oralen, rektalen, parenteralen oder topischen Anwendung sind im Handel.

Diclofenac (Formel 2) hat sich aufgrund der wirksamen Dosis und der totalen Clearance als das potenteste nicht-steroidale Antiinflammatorikum/Analgetikum erwiesen.

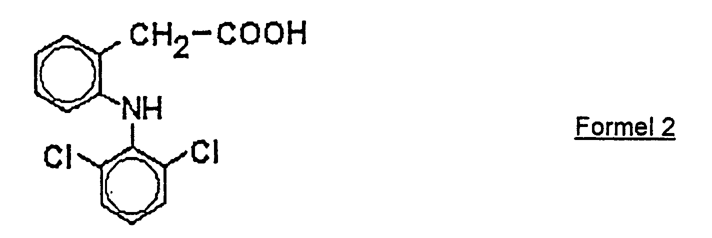

Bei peroraler Anwendung ist Diclofenac (Formel 2) klinisch pharmakologisch im Dosisbereich von 0,08 bis 0,16 mmol/8 Std. wirksam und liegt damit im Bereich der molaren Dosierung von Morphin (Formel 3a). Zum Vergleich: Morphin ist im Dosisbereich von 0,035 bis 0,35 mmol/6 Std. wirksam.

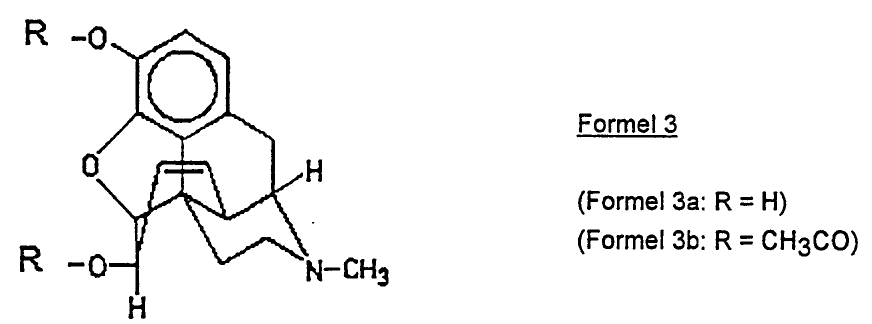

In der Formel 3 kann R = H (Morphin) oder CH₃CO (Diamorphin) bedeuten.

Die erfindungsgemäßen Salze, zusammengesetzt aus der Base des narkotischen Analgetikums der Formel 3, in der R für H oder CH₃CO steht, und der Säure des nicht-narkotischen Analgetikums vom Typ der substituierten Carbonsäuren, vorzugsweise das Morphin- oder Diamorphin-Diclofenat (Formel 1a oder 1b), ermöglichen/ermöglicht es vorteilhafterweise, die Teilkomponenten in geringerer Dosierung einzusetzen als die Zubereitungen der Monosubstanzen in Form ihrer anorganischen Salze in freier Kombination der herkömmlichen kommerziellen Darreichungsformen.

Die erfindungsgemäßen Salze, vorzugsweise das Morphin- oder Diamorphin-Diclofenat (Formel 1a oder 1b) können/kann anstelle der klassischen Morphinpräparate, jedoch mit geringeren Nebenwirkungen, zur Schmerzlinderung eingesetzt werden. Im Fall der erfindungsgemäßen Diclofenate sind die Grenzen der gastrointestinalen Verträglichkeit des Diclofenac (Formel 2) eine Sicherung zur Einhaltung der offiziellen Dosierungsempfehlung. Die erfindungsgemäßen Morphin- oder Diamorphin-Salze erschweren folglich den potentiellen Medikamentenmißbrauch, der der Verfügbarkeit von Morphinpräparaten anhaftet. Die erfindungsgemäßen Morphin- oder Diamorphin-Salze erlauben dem Arzt, den Patienten besser in ein Behandlungsschema einzubeziehen, da weniger Medikamente in freien Kombinationen und weniger laienhafte Abwandlungen der ärztlichen Empfehlung möglich sind.

Die erfindungsgemäßen Diamorphin-Salze ermöglichen es erstmals, Diamorphin in die Therapie einzubeziehen.

Die topischen Zubereitungen der erfindungsgemäßen Salze, insbesondere die des Morphin-Diclofenat (Formel 1a) und des Diamorphin-Diclofenat (Formel 1b) erlauben, beispielsweise in der Entwöhnungsphase der Suchttherapie, die minimal nötige Wirkstoffzufuhr unter verminderten systemischen Nebenwirkungen.

Hergestellt werden die erfindungsgemäßen Wirkstoffe aus den an sich bekannten Säure- bzw, Basenkomponenten. Bevorzugt werden nicht-narkotische Analgetika vom Typ der substituierten Carbonsäuren, insbesondere die [2-[(2,6-Dichlorphenyl)-amino]-phenyl]-essigsäure der Formel 2 oder ein Basensalz davon mit vorzugsweise der mindestens äquimolaren Menge Morphin (Formel 3a) oder dem Morphinderivat der Formel 3b oder einem geeigneten Salz hiervon umgesetzt. Verwendbare Salze der nicht-narkotischen Analgetika vom Typ der substituierten Carbonsäuren sind insbesondere Salze von Basen, die aus dem Reaktionsgemisch leicht entfernt werden können. Hierzu gehören beispielsweise Basen, die flüchtiger oder schwächer sind als Morphin oder Diamorphin (Formel 3a oder 3b), oder die mit dem anionisch nicht-narkotischen Analgetikum vom Typ der substituierten Carbonsäuren, insbesondere mit der [2-[(2,6-Dichlorphenyl)-amino]-phenyl]-essigsäure der Formel 2 leichter lösliche Salze bilden als die erfindungsgemäßen Salze. Solche Salze sind beispielsweise andere organische Ammoniumsalze. Ebenfalls verwendbare Salze sind Metallsalze, die bei der Umsetzung mit einem geeigneten Säuresalz des Morphin oder Diamorphin (Formel 3a oder 3b) schwerlösliche Salze eben dieser Säure bilden, z.B. Calciumsalze.

Verfahrensgemäß einsetzbare Salze des Morphin oder Diamorphin (Formel 3a oder 3b) sind beispielsweise Salze mit Säuren, die aus dem Reaktionsgemisch entfernt werden können, beispielsweise Salze von flüchtigen Säuren. Es können auch Säuren eingesetzt werden, die schwächer sind als das nicht-narkotische Analgetikum vom Typ der substituierten Carbonsäuren oder die mit Metallkationen, beispielsweise Ca²⁺, schwerlösliche Salze bilden. Salze des Morphin oder Diamorphin (Formel 3a oder 3b) sind beispielsweise Salze der Verbindungen gemäß Formel 3a oder 3b mit anorganischen Säuren, z.B. Hydrochloride, Sulfate oder Phosphate oder Salze aus Morphin oder Diamorphin (Formel 3a oder 3b) und organischen Säuren, wie z.B. Fumarate, Maleate oder Oxalate.

Die Reaktion der nicht-narkotischen Analgetika vom Typ der substituierten Carbonsäuren, vorzugsweise der [2-[(2,6-Dichlorphenyl)-amino]-phenyl]-essigsäure (Formel 2), mit einer Base der Formel 3 erfolgt vorzugsweise in einem inerten Lösungsmittel. Falls erforderlich, kann die Reaktion unter Kühlen oder Erwärmen, beispielsweise in einem Temperaturbereich von etwa 0 bis etwa 100°C, vorzugsweise bei Raumtemperatur, durchgeführt werden. Die Umsetzung kann in einem geschlossenen Gefäß und/oder unter Inertgasatmosphäre, z.B. in Stickstoffatmosphäre erfolgen.

Als inerte Lösungsmittel können beispielsweise Alkohole, Ether, Ketone, Carbonsäureester, Amide, Sulfoxide, chlorierte Kohlenwasserstoffe oder Gemische dieser Lösungsmittel Verwendung finden. Als Alkohole einsetzbar sind Niederalkanole, vorzugsweise Methanol oder Ethanol, als Ether Diniederalkylether, vorzugsweise Diethylether, cyclische Ether, vorzugsweise Dioxan oder Tetrahydrofuran, als Ketone Diniederalkylketone, vorzugsweise Aceton, als Carbonsäureester Niederalkylcarbonsäureester, vorzugsweise Essigsäureethylester, als Amide N,N-Diniederalkylamide, vorzugsweise N,N-Dimethylformamid, als Sulfoxide Diniederalkylsulfoxide, vorzugsweise Dimethylsulfoxid, und als chlorierte Kohlenwasserstoffe Methylenchlorid oder Chloroform.

Nicht-narkotische Analgetika vom Typ der substituierten Carbonsäuren, insbesondere die [2-[(2,6-Dichlorphenyl)-amino]-phenyl]-essigsäure (Formel 2) können/kann auch unter den Reaktionsbedingungen aus den entsprechenden Estern, vorzugsweise Niederalkylester, durch Hydrolyse in Gegenwart einer Base, beispielsweise NaOH, gebildet werden. Das Morphin oder Diamorphin der Formel 3a oder 3b kann ebenfalls unter den Reaktionsbedingungen aus den Säuresalzen, vorzugsweise aus den Hydrochloriden, Sulfaten oder Phosphaten in Gegenwart stöchiometrischer Mengen geeigneter Basen, beispielsweise NaOH, freigesetzt werden.

In einer bevorzugten Ausführung des Verfahrens wird das nicht-narkotische Analgetikum vom Typ der substituierten Carbonsäuren als freie Säure gemäß Formel 2 in einem geeigneten Lösungsmittel direkt mit dem Morphin oder Diamorphin der Formel 3a oder 3b als Base umgesetzt. Die Erfindung betrifft auch diejenigen Herstellungsverfahren, bei denen die Ausgangsstoffe in situ hergestellt werden oder bei denen ein Ausgangsstoff unter den Reaktionsbedingungen aus einem Derivat gewonnen wird und/oder in Form eines Stoffgemisches, z.B. im Fall des Morphin oder Diamorphin (Formel 3a oder 3b) als Rohalkaloidgemisch, eingesetzt wird. Erfindungsgemäß sind auch Verfahren, in denen flüssige oder feste geladene oder nicht-geladene anorganische oder organische Adsorbentien eingesetzt werden. So können beispielsweise lonenaustauscher eingesetzt werden, um die kationische oder anionische Komponente der erfindungsgemäßen Salze zu binden, die anschließend mit der komplementären anionischen bzw. kationischen Komponente umgesetzt werden. Nicht-geladene anorganische oder organische Adsorbentien zur Präparation oder Reinigung der erfindungsgemäßen Salze sind ebenfalls einsetzbar.

Die Erfindung betrifft weiterhin pharmazeutische Präparate, die neben an sich bekannten pharmazeutischen Hilfsstoffen die Salze aus Morphin (Formel 3a) oder Diacetylmorphin (Formel 3b) und analgetisch wirksamen Carbonsäuren, insbesondere die Salze der Formel 1a und 1b, Morphin-Diclofenat oder Diamorphin-Diclofenat, enthalten, sowie Verfahren zur Herstellung solcher pharmazeutischen Präparate. Die erfindungsgemäßen pharmazeutischen Präparate betreffen solche zur enteralen, beispielsweise oralen oder rektalen Verabreichung, zur parenteralen, beispielsweise intravenösen, intramuskulären oder subkutanen Anwendung oder zur topischen Applikation, die die erfindungsgemäßen Salze, insbesondere die Salze der Formel 1a und 1b, allein oder zusammen mit pharmazeutisch anwendbaren Trägermaterialien, insbesondere geeignet zur kontrollierten Wirkstoff-Freisetzung, enthalten.

In Dosis-Einheitsformen für die perorale Anwendung liegt der Gehalt des erfindungsgemäßen Wirkstoffs vorzugsweise zwischen 10 und 90%. Zur Herstellung von Tabletten oder Dragée-Kernen wird der Wirkstoff z.B. mit festen pulverförmigen Trägerstoffen, wie Lactose, Saccharose, Sorbit, Mannit, Stärken oder Amylopektin, ferner Cellulosederivaten, Gelatine oder Polyvinylpyrrolidon, gegebenenfalls unter Zusatz von Gleitmitteln, wie Magnesium- oder Calciumstearat oder Polyethylenglykolen sowie mit hochdisperser Kieselsäure kombiniert. Dragée-Kerne werden beispielsweise mit konzentrierten Zuckerlösungen, die gegebenenfalls noch Zusatzstoffe wie z.B. Gummi arabicum, Talk und/oder Titandioxid enthalten können oder mit einem in leichtflüchtigen organischen Lösungsmitteln oder Lösungsmittelgemischen gelöstem Lack, überzogen. Diesen Überzügen können Farbstoffe zugefügt werden, z.B. zur Kennzeichnung verschiedener Wirkstoffdosen. Als orale Darreichungsformen eignen sich ferner Steckkapseln aus Gelatine sowie weiche geschlossene Kapseln aus Gelatine und einem Weichmacher wie Glycerin. Die ersteren enthalten den erfindungsgemäßen Wirkstoff vorzugsweise als Granulat in Mischung mit Gleitmitteln, wie Talk oder Magnesiumstearat und gegebenenfalls Stabilisatoren wie Natriumpyrosulfit (Na₂S₂O₅) oder Ascorbinsäure. In weichen Kapseln ist der erfindungsgemäße Wirkstoff vorzugsweise in geeigneten Flüssigkeiten, wie flüssigen Polyethylenglykolen, gelöst oder suspendiert, wobei ebenfalls Stabilisatoren zugefügt sein können. Ferner können die erfindungsgemäßen Wirksubstanzen zur kontrollierten Freisetzung in eine pharmazeutisch akzeptable Trägermatrix eingearbeitet sein oder in einem membranbegrenztem Reservoir, das sich zur zweckentsprechenden Freisetzungskinetik in vivo aktiviert, eingeschlossen sein.

Als Dosis-Einheitsformen für die rektale Anwendung kommen z.B. Suppositorien in Betracht, die aus dem erfindungsgemäßen Wirkstoffsalz in einer Suppositoriengrundmasse auf der Basis von natürlichen oder synthetischen Triglyceriden von geeignetem Schmelzpunkt, z.B. Kakaobutter, oder auf der Basis von Polyethylenglykolen oder geeigneten höheren Fettalkoholen bestehen.

Zur parenteralen Verabreichung eignen sich vorzugsweise Lösungen des erfindungsgemäßen Wirkstoffes, ferner dessen Suspensionen, wie entsprechende ölige Injektionssuspensionen, wobei geeignete lipophile Lösungsmittel oder Vehikel, wie Öle, z.B. Sesamöl,oder synthetische Fettsäureester, z.B. Ethyloleat oder Triglyceride, oder wobei wäßrige Injektionssuspensionen, die viskositätserhöhende Stoffe, z.B. Natriumcarboxymethylcellulose, Sorbit und/oder Dextran und gegebenenfalls auch Stabilisatoren enthalten, verwendet werden.

Topisch anwendbare pharmazeutische Präparate der erfindungsgemäßen Salze, vorzugsweise des Morphin-Diclofenat (Formel 1a) oder Diamorphin-Diclofenat (Formel 1b) sind Cremes, Salben, Gele, Pasten, Schäume, Tinkturen und Lösungen, die etwa 0,5 bis etwa 20% des erfindungsgemäßen Wirkstoffes enthalten. Ein erfindungsgemäßes Salz, vorzugsweise das Morphin-Diclofenat (Formel 1a) oder Diamorphin-Diclofenat (Formel 1b) kann auch in Pflastern oder sogenannten transdermalen therapeutischen Systemen (TTS) eingearbeitet sein, aus denen die Wirkstoffkomponenten über einer definierten Fläche der Körperoberhaut in okklusiver Weise mit kontrollierter Abgabegeschwindigkeit auf die Haut einwirken oder zweckentsprechend zur transdermalen Resorption gebracht werden.

Die nachfolgenden Beispiele sollen die Erfindung erläutern:

### Herstellungsverfahren der Salze

### Beispiel 1 :

Zu einer wässrigen Lösung von Morphinhydrochlorid wird eine äquimolare Menge einer wässrigen Lösung von Diclofenac-Natriumsalz gegeben und durch Rühren und gegebenenfalls durch Erhitzen in Lösung gebracht. Der sich bildende Niederschlag aus Morphindiclofenat wird abgenutscht und im Exsikkator über Molekularsieb getrocknet.

IR-spekroskopisch läßt sich in der kristallinen Substanz sowohl der Morphin- als auch der Diclofenac-Anteil nachweisen.

Der Schmelzpunkt des Salzes liegt bei 143°C und unterscheidet sich damit deutlich von denen der Ausgangssubstanzen (Morphinhydrochlorid: 255°C; Diclofenac-Natriumsalz: 280°C).

### Beispiel 2 :

Äquimolare Mengen der Morphinbase bzw. der Diamorphinbase und der [2-[(2,6-Dichlorphenyl)-amino]-phenyl]-essigsäure werden in Ethanol gelöst, im Rundkolben gemischt und am Rotationsverdampfer zur Trockne eingedampft. Der Eindampfrückstand wird aus Ethanol unter Zusatz von Petrolether umkristallisiert, abgenutscht und im Vakuum getrocknet.

### Beispiel 3 :

1 Millimol Morphinhydrogensulfat bzw. Diamorphinhydrogensulfat wird in Wasser gelöst, mit wässriger Natriumbicarbonatlösung alkalisch gestellt und mit Essigester extrahiert. Ebenso wird 1 Millimol Diclofenac-Natrium in Wasser gelöst, mit verdünnter Salzsäure angesäuert und mit Essigester extrahiert. Die beiden Extrakte werden separat über wasserfreiem Natriumsulfat getrocknet. Anschließend werden die beiden Extrakte vereinigt, gemischt und im Rundkolben am Rotationsverdampfer zur Trockne eingedampft. Der Eindampfrückstand wird aus Ethanol unter Zusatz von Petrolether umkristallisiert und im Vakuum getrocknet.

### Herstellungsverfahren eines pharmazeutischen Präparats

### Beispiel 4:

Zur peroralen Verabreichung wird Morphin-Diclofenat bzw. Diamorphin-Diclofenat fein vermahlen und bis zur vierfachen Menge mit pulverförmigen Trägerstoffen wie Laktose, Saccharose, Sorbit, Mannit oder Stärken homogen vermischt. Einzeldosen von beispielsweise 0,05 Millimol Wirkstoffäquivalenten werden in Gelatinekapseln eingewogen.

## Patentansprüche

1. Salz aus einem kationischen narkotischen Analgetikum mit einem anionischen nicht-narkotischen Analgetikum, **dadurch gekennzeichnet, daß** die Base des narkotischen Analgetikums der Formel 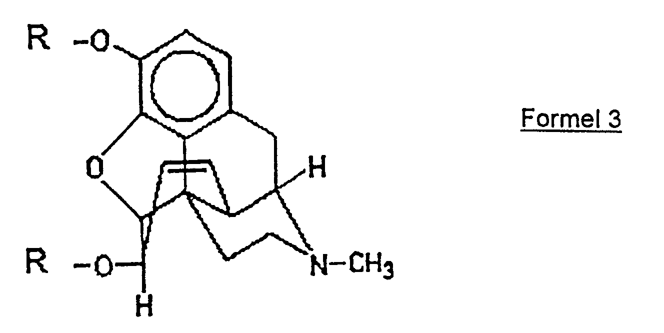 entspricht, wobei R für H oder CH₃CO steht und das nicht-narkotische Analgetikum zum Typ der substituierten Carbonsäuren gehört.

2. Salz gemäß Anspruch 1, **dadurch gekennzeichnet, daß** das Anion des nicht-narkotischen Analgetikums vom Typ der substituierten Carbonsäuren Diclofenac, Indometacin, Sulindac, Ketoprofen oder Fenbufen, vorzugsweise Diclofenac ist.

3. Verfahren zur Herstellung des Salzes gemäß Anspruch 1 oder 2, **dadurch gekennzeichnet, daß** die Base des narkotischen Analgetikums der Formel 3 entspricht, in der R für H oder CH₃CO steht oder einem geeigneten Salz hiervon, mit der Säure des nicht-narkotischen Analgetikums vom Typ der substituierten Carbonsäuren oder einem Basensalz davon in einem inerten Lösungsmittel in einem Temperaturbereich von 0 bis 100°C umgesetzt wird.

4. Verfahren gemäß Anspruch 3, **dadurch gekennzeichnet, daß** ein Salz aus dem nicht-narkotischen Analgetikum vom Typ der substituierten Carbonsäuren mit Basen, die flüchtiger oder schwächer sind als die Base des narkotischen Analgetikums der Formel 3, in der R für H oder CH₃CO steht oder ein Salz aus dem nicht-narkotischen Analgetikum vom Typ der substituierten Carbonsäuren mit Basen, die mit dem nicht-narkotischen Analgetikum vom Typ der substituierten Carbonsäuren Salze bilden, die leichter löslich sind als das Salz gemäß Anspruch 1, eingesetzt wird.

5. Verfahren gemäß Anspruch 4, **dadurch gekennzeichnet, daß** als Salz ein organisches Ammoniumsalz oder ein Metallsalz, vorzugsweise ein Calciumsalz, eingesetzt wird.

6. Verfahren gemäß einem der Ansprüche 3 bis 5, **dadurch gekennzeichnet, daß** ein Salz aus der Base des narkotischen Analgetikums der Formel 3, in der R für H oder CH₃CO steht, mit Säuren, die flüchtiger oder schwächer sind als die Säure des nicht-narkotischen Analgetikums vom Typ der substituierten Carbonsäuren oder ein Salz aus der Base des narkotischen Analgetikums der Formel 3, in der R für H oder CH₃CO steht, mit Säuren, die mit Metallkationen, vorzugsweise mit Ca^{2+,} schwerlösliche Salze bilden, eingesetzt wird.

7. Verfahren gemäß Anspruch 6, **dadurch gekennzeichnet, daß** ein Salz aus der Base des narkotischen Analgetikums der Formel 3, in der R für H oder CH₃CO steht, mit anorganischen Säuren, vorzugsweise ein Hydrochlorid, Sulfat oder Phosphat oder ein Salz aus der Base des narkotischen Analgetikums der Formel 3, in der R für H oder CH₃CO steht, mit organischen Säuren, vorzugsweise ein Fumarat, Maleat oder ein Oxalat eingesetzt wird.

8. Verfahren gemäß einem der Ansprüche 3 bis 7, **dadurch gekennzeichnet, daß** als Säure des nicht-narkotischen Analgetikums vom Typ der substituierten Carbonsäuren Diclofenac ([2-[(2,6-Dichlorphenyl)-amino]-phenyl]-essigsäure) eingesetzt wird.

9. Verfahren zur Herstellung des Salzes gemäß Anspruch 1 oder 2, **dadurch gekennzeichnet, daß** ein Säuresalz des basischen narkotischen Analgetikums der Formel 3, in der R für H oder CH₃CO steht, vorzugsweise das Hydrochlorid, Sulfat oder Phosphat, mit einem Ester des nicht-narkotischen Analgetikums vom Typ der substituierten Carbonsäuren, vorzugsweise einem Niederalkylester, in Gegenwart einer Base, vorzugsweise NaOH in einem inerten Lösungsmittel in einem Temperaturbereich von 0 bis 100°C umgesetzt wird.

10. Verfahren zur Herstellung des Salzes gemäß Anspruch 1 oder 2, **dadurch gekennzeichnet, daß** die Base des narkotischen Analgetikums der Formel 3, in der R für H oder CH₃CO steht, mit der Säure des nicht-narkotischen Analgetikums vom Typ der substituierten Carbonsäuren in einem inerten Lösungsmittel in einem Temperaturbereich von 0 bis 100°C umgesetzt wird.

11. Verfahren gemäß einem der Ansprüche 9 oder 10, **dadurch gekennzeichnet, daß** die Base des narkotischen Analgetikums gemäß Formel 3, in der R für H oder CH₃CO steht, als Rohalkaloidextrakt im Gemisch eingesetzt wird.

12. Verfahren zur Herstellung des Salzes gemäß Anspruch 1 oder 2, **dadurch gekennzeichnet, daß** das Kation bzw. die Base des narkotischen Analgetikums der Formel 3, in der R für H oder CH₃CO steht oder das Anion bzw. die Säure des nicht-narkotischen Analgetikums vom Typ der substituierten Carbonsäuren an flüssige oder feste geladene oder nicht-geladene anorganische oder organische Adsorbentien, vorzugsweise lonenaustauscher, gebunden wird und mit der jeweils komplementären anionischen bzw. kationischen Komponente in einem inerten Lösungsmittel in einem Temperaturbereich von 0 bis 100°C umgesetzt wird.

13. Verfahren gemäß einem der Ansprüche 3 bis 12, **dadurch gekennzeichnet, daß** zumindest äquimolare Mengen der Reaktionspartner eingesetzt werden.

14. Verfahren gemäß einem der Ansprüche 3 bis 13, **dadurch gekennzeichnet, daß** die Umsetzung bei Raumtemperatur und/oder unter Inertgasatmosphäre, vorzugsweise unter Stickstoffatmosphäre, durchgeführt wird.

15. Verfahren gemäß einem der Ansprüche 3 bis 14, **dadurch gekennzeichnet, daß** als inerte Lösungsmittel Alkohole, Ether, Ketone, Carbonsäureester, Amide, Sulfoxide, chlorierte Kohlenwasserstoffe oder Gemische dieser Lösungsmittel eingesetzt werden.

16. Verfahren gemäß einem der Ansprüche 3 bis 15, **dadurch gekennzeichnet, daß** als Alkohole Niederalkanole, vorzugsweise Methanol oder Ethanol, als Ether Diniederalkylether oder cyclische Ether, vorzugsweise Diethylether, Dioxan oder Tetrahydrofuran, als Ketone Diniederalkylketone, vorzugsweise Aceton, als Carbonsäureester Niederalkylcarbonsäureester, vorzugsweise Essigsäureethylester, als Amide N,N-Diniederalkylamide, vorzugsweise N,N-Dimethylformamid, als Sulfoxide Diniederalkylsulfoxide, vorzugsweise Dimethylsulfoxid, und als chlorierte Kohlenwasserstoffe vorzugsweise Methylenchlorid oder Chloroform oder Gemische dieser Lösungsmittel eingesetzt werden/wird.

17. Pharmazeutisches Präparat, **dadurch gekennzeichnet, daß** es neben den an sich bekannten pharmazeutischen Hilfsstoffen ein Salz gemäß Anspruch 1 oder 2 enthält.

18. Pharmazeutisches Präparat gemäß Anspruch 17 zur enteralen, beispielsweise oralen oder rektalen Applikation oder zur parenteralen, beispielsweise intravenösen, intramuskulären, subkutanen oder topischen Applikation

19. Verwendung des Salzes gemäß Anspruch 1 oder 2 zur Herstellung galenischer Zubereitungsformen.

20. Salz gemäß Anspruch 1 oder 2 zur therapeutischen Behandlung des menschlichen oder tierischen Organismus.

21. Salz gemäß Anspruch 1 oder 2 zur therapeutischen Behandlung von Schmerzen.

22. Pharmazeutisches Präparat gemäß Anspruch 17 oder 18 zur therapeutischen Behandlung des menschlichen oder tierischen Organismus.

23. Pharmazeutisches Präparat gemäß Anspruch 17 oder 18 zur therapeutischen Behandlung von Schmerzen.

24. Verfahren zur Herstellung eines Miltels zur therapeutischen Behandlung des menschlichen oder tierischen Organismus, **dadurch gekennzeichnet, daß** ein Salz gemäß einem der Ansprüche 1 oder 2 oder ein pharmazeutisches Präparat gemäß einem der Ansprüche 17 oder 18 eingesetzt wird.

25. Verfahren zur Herstellung eines Mittels zur Behandlung von Schmerzen, **dadurch gekennzeichnet, daß** ein Salz gemäß einem der Ansprüche 1 oder 2 oder ein pharmazeutisches Präparat gemäß einem der Ansprüche 17 oder 18 eingesetzt wird.

## Claims

1. A salt of a cationic narcotic analgesic with an anionic non-narcotic acid **characterized in that** the base of the narcotic analgesic corresponds to the formula 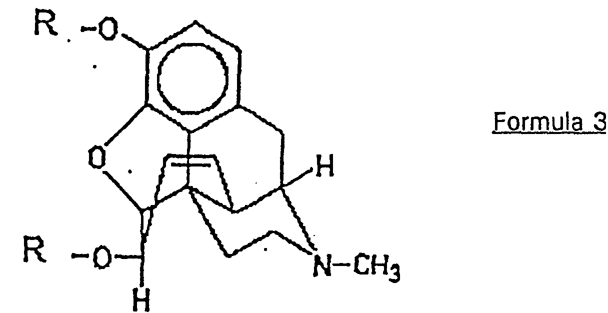 wherein R stands for H or CH₃CO, and the non-narcotic analgesic belongs to the type of substituted carboxylic acids.

2. The salt according to claim 1 **characterized in that** the anion of the non-narcotic analgesic of the type substituted carboxylic acids is diclofenac, indomethacin, sulindac, ketoprofen, or fenbufen, preferably is diclofenac.

3. A process for the production of the salt according to claim 1 or 2 **characterized in that** the base of the narcotic analgesic corresponds to formula 3, wherein R stands for H or CH₃CO or a suitable salt thereof, and is reacted with the acid of the non-narcotic analgesic of the type substituted carboxylic acids or with a base salt thereof in an inert solvent at a temperature ranging from 0 to 100°C.

4. The process according to claim 3 **characterized in that** a salt of the non-narcotic analgesic of the type substituted carboxylic acids is used with bases which are more volatile or weaker than the base of the narcotic analgesic of formula 3, wherein R stands for H or CH₃CO, or that a salt of the non-narcotic analgesic of the type substituted carboxylic acids is used with bases which form salts with the non-narcotic analgesic of the type substituted carboxylic acids, which are more readily soluble than the salt according to claim 1.

5. The process according to claim 4 **characterized in that** an organic ammonium salt or a metallic salt, preferably a calcium salt, is used as salt.

6. The process according to any one of claims 3 to 5 **characterized in that** a salt from the base of the narcotic analgesic of formula 3, wherein R stands for H or CH₃CO, is used with acids which are more volatile or weaker than the acid of the non-narcotic analgesic of the type substituted carboxylic acids, or that a salt from the base of the narcotic analgesic of formula 3, wherein R stands for H or CH₃CO, is used with acids which form poorly soluble salts with metal cations, preferably with Ca²⁺.

7. The process according to claim 6 **characterized in that** a salt from the base of the narcotic analgesic of formula 3, wherein R stands for H or CH₃CO, is used with inorganic acids, preferably a hydrochloride, sulfate, or phosphate, or that a salt from the base of the narcotic analgesic of formula 3, wherein R stands for H or CH₃CO, is used with organic acids, preferably a fumarate, maleate, or oxalate.

8. The process according any one of claims 3 to 7 **characterized in that** diclofenac ([2-[(2,6-dichlorophenyl)-amino]-phenyl]-acetic acid) is used as the acid of the non-narcotic analgesic of the type substituted carboxylic acids.

9. A process for the production of a salt according to claim 1 or 2 **characterized in that** an acid salt of the basic narcotic analgesic of formula 3, wherein R stands for H or CH₃CO, preferably the hydrochloride, sulfate, or phosphate, is reacted with an ester of the non-narcotic analgesic of the type substituted carboxylic acids, preferably a low-alkyl ester, in the presence of a base, preferably NaOH, in an inert solvent at a temperature ranging from 0 to 100°C.

10. A process for the production of the salt according to claim 1 or 2 **characterized in that** the base of the narcotic analgesic of formula 3, wherein R stands for H or CH₃CO, is reacted with the acid of the non-narcotic analgesic of the type substituted carboxylic acids in an inert solvent at a temperature ranging from 0 to 100°C.

11. The process according to any one of claims 9 or 10 **characterized in that** the base of the narcotic analgesic of formula 3, wherein R stands for H or CH₃CO, is used as raw alkaloid extract in admixture.

12. A process for the production of the salt according to claim 1 or 2 **characterized in that** the cation or the base of the narcotic analgesic of formula 3, wherein R stands for H or CH₃CO, or the anion or the acid of the non-narcotic analgesic of the type substituted carboxylic acids is bound to liquid or solid charged or uncharged inorganic or organic adsorbents, preferably ion exchangers, and is reacted with the respective complementary anionic or cationic component in an inert solvent at a temperature ranging form 0 to 100°C.

13. The process according to any one of claims 3 to 12 **characterized in that** at least equimolar amounts of reactants are used.

14. The process according to any one of claims 3 to 13 **characterized in that** the reaction is carried out at room temperature and/or under inert gas atmosphere, preferably under nitrogen atmosphere.

15. The process according to any one of claims 3 to 14 **characterized in that** alcohols, ethers, ketones, carboxylic acid esters, amides, sulfoxides, chlorinated hydrocarbons, or mixtures of the these solvents are used as inert solvents.

16. The process according to any one of claims 3 to 15 **characterized in that** low alkanols, preferably methanol or ethanol are used as alcohols; di-low-alkyl ethers or cyclic ethers, preferably diethyl ether, dioxan or tetrahydrofuran are used as ethers; di-low-alkyl ketones, preferably acetone are used as ketones; low-alkyl carboxylic acid esters, preferably acetic acid ethyl ester are used as carboxylic acid esters; N,N-di-low-alkyl amides, preferably N,N-dimethylformamide are used as amides; di-low-alkyl sulfoxides, preferably dimethyl sulfoxide are used as sulfoxides; and methylene chloride or chloroform are preferably used as chlorinated hydrocarbons, or that mixtures of these solvents are used.

17. A pharmaceutical preparation **characterized in that** it comprises a salt according to claim 1 or 2 in addition to pharmaceutical adjuvants known per se.

18. The pharmaceutical preparation according to claim 17 for enteral, for example, oral or rectal administration, or for parenteral, for example, intravenous, intramuscular, subcutaneous, or topical application.

19. The use of the salt according to claim 1 or 2 for the production of galenic forms of preparation.

20. The salt according to claim 1 or 2 for the therapeutic treatment of human and animal organisms.

21. The salt according to claim 1 or 2 for the therapeutic treatment of pain.

22. The pharmaceutical preparation according to claim 17 or 18 for the therapeutic treatment of human and animal organisms.

23. The pharmaceutical preparation according to claim 17 or 18 for the therapeutic treatment of pain.

24. A process for the production of a preparation for the therapeutic treatment of human or animal organisms **characterized in that** a salt according to any one of claims 1 or 2 or a pharmaceutical preparation according to any one of claims 17 or 18 is used.

25. A process for the production of a preparation for the treatment of pain **characterized in that** a salt according to any one of claims 1 or 2 or a pharmaceutical preparation according to any one of claims 17 or 18 is used.

## Revendications

1. Sel d'un analgésique narcotique cationique avec un analgésique non narcotique anionique, **caractérisé en ce que** la base de l'analgésique narcotique correspond à la formule 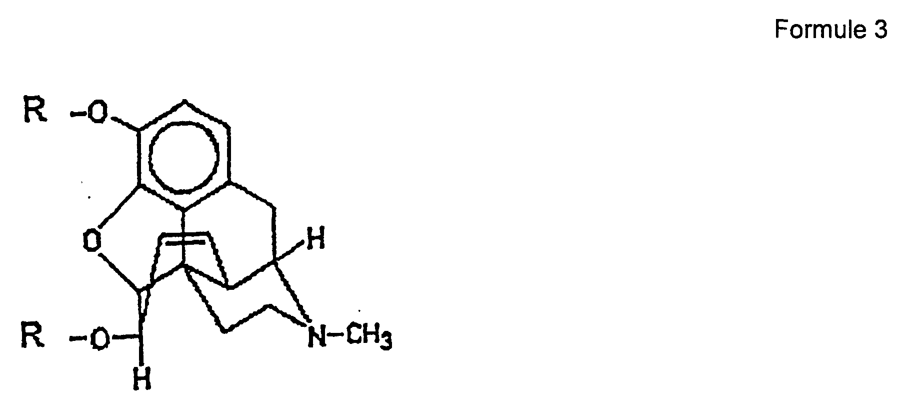 dans laquelle R représente H ou CH₃CO et l'analgésique non narcotique est du type acide carboxylique substitué.

2. Sel selon la revendication 1, **caractérisé en ce que** l'anion de l'analgésique non narcotique de type acide carboxylique substitué est le diclophenac, l'indométacine, le sulindac, le cetoprofen ou le fenbufen, de préférence le diclophenac.

3. Procédé de préparation du sel selon la revendication 1 ou 2, **caractérisé en ce que** l'on fait réagir la base de l'analgésique narcotique correspondant à la formule 3 dans laquelle R représente H ou CH₃CO ou à un sel approprié de cette base avec l'acide de l'analgésique non narcotique de type acide carboxylique substitué ou avec un sel basique de ce dernier dans un solvant inerte, dans une plage de température de 0 à 100°C.

4. Procédé selon la revendication 3, **caractérisé en ce que** l'on fait réagir un sel de l'analgésique non narcotique du type acide carboxylique substitué avec des bases qui sont plus volatiles ou plus faibles que la base de l'analgésique narcotique de formule 3 dans laquelle R représente H ou CH₃CO, ou un sel de l'analgésique non narcotique du type acide carboxylique substitué et de bases qui forment des sels avec l'analgésique non narcotique du type acide carboxylique substitué et qui sont plus aisément solubles que le sel selon la revendication 1.

5. Procédé selon la revendication 4, **caractérisé en ce qu'**on utilise comme sel un sel organique d'ammonium ou un sel métallique, de préférence un sel de calcium.

6. Procédé selon l'une des revendications 3 à 5, **caractérisé en ce que** l'on utilise un sel obtenu à partir de la base de l'analgésique narcotique de formule 3 dans laquelle R représente H ou CH₃CO, et d'acides qui sont plus volatils ou plus faibles que l'acide de l'analgésique non narcotique de type acide carboxylique substitué, ou un sel obtenu à partir de la base de l'analgésique narcotique de formule 3 dans laquelle R représente H ou CH₃CO, et d'acides qui forment des sels difficilement solubles avec des cations métalliques, de préférence avec CA²⁺.

7. Procédé selon la revendication 6, **caractérisé en ce que** l'on utilise un sel obtenu à partir de la base de l'analgésique narcotique de formule 3 dans laquelle R représente H ou CH₃CO avec des acides minéraux, de préférence un chlorhydrate, un sulfate ou un phosphate, ou un sel obtenu à partir de la base de l'analgésique narcotique de formule 3 dans laquelle R représente H ou CH₃CO et avec des acides organiques, de préférence un fumarate, un maléate ou un oxalate.

8. Procédé selon l'une des revendications 3 à 7, **caractérisé en ce que** l'on utilise comme acide de l'analgésique non narcotique du type acide carboxylique substitué le diclophenac (acide [2-[2,6-dichlorophényl)amino]phényl]acétique).

9. Procédé pour la préparation du sel selon les revendications 1 ou 2, **caractérisé en ce que** l'on fait réagir un sel d'acide de l'analgésique narcotique basique de formule 3 dans laquelle R représente H ou CH₃CO, de préférence le chlorhydrate, le sulfate ou le phosphate, avec un ester de l'analgésique non narcotique du type acide carboxylique substitué, de préférence un ester d'alkyle inférieur, en présence d'une base, de préférence NaOH, dans un solvant inerte et dans une plage de température de 0 à 100°C.

10. Procédé pour la préparation du sel selon les revendications 1 ou 2, **caractérisé en ce que** l'on fait réagir la base de l'analgésique narcotique de formule 3 dans laquelle R représente H ou CH₃CO avec l'acide de l'analgésique non narcotique du type acide carboxylique substitué dans un solvant inerte, dans une plage de température de 0 à 100°C.

11. Procédé selon l'une des revendications 9 ou 10, **caractérisé en ce que** l'on utilise la base de l'analgésique narcotique selon la formule 3 dans laquelle R représente H ou CH₃CO en tant qu'extrait brut d'alcaloïdes en mélange.

12. Procédé pour la préparation du sel selon la revendication 1 ou 2, **caractérisé en ce que** l'on fait réagir le cation ou la base de l'analgésique narcotique de formule 3 dans laquelle R représente H ou CH₃CO ou l'anion resp. l'acide de l'analgésique non narcotique du type acide carboxylique substitué sur des adsorbants minéraux ou organiques liquides ou solides, chargés ou non chargés, de préférence des échangeurs d'ions, en les y liant, et on les fait réagir avec le composant anionique resp. cationique complémentaire dans un solvant inerte, dans une plage de température de 0 à 100°C.

13. Procédé selon l'une des revendications 3 à 12, **caractérisé en ce que** l'on utilise des quantités au moins équimolaires des partenaires de réaction.

14. Procédé selon l'une des revendications 3 à 13, **caractérisé en ce que** l'on réalise la réaction à température ambiante et/ou sous une atmosphère de gaz inerte, de préférence sous une atmosphère d'azote.

15. Procédé selon l'une des revendications 3 à 14, **caractérisé en ce que** l'on utilise comme solvant inerte des alcools, des éthers, des cétones, des esters d'acide carboxylique, des amides, des sulfoxydes, des hydrocarbures chlorés ou des mélanges de ces solvants.

16. Procédé selon l'une des revendications 3 à 15, **caractérisé en ce que** l'on utilise comme alcools des alcools inférieurs, de préférence le méthanol ou l'éthanol, comme éthers des diéthers d'alkyle inférieur ou des éthers cycliques, de préférence l'éther de diéthyle, le dioxane et le tétrahydrofurane, comme cétones des cétones de di(alkyle inférieur), de préférence l'acétone, comme ester d'acide carboxylique un ester d'acide carboxylique et d'alkyle inférieur, de préférence un éthylester d'acide acétique, comme amides des amides de di(alkyle inférieur), de préférence le N,N-diméthylformamide, comme sulfoxydes des sulfoxydes de di(alkyle inférieur), de préférence le sulfoxyde de diméthyle et comme hydrocarbures chlorés, de préférence le chlorure de méthylène ou le chloroforme, ou des mélanges de ces solvants.

17. Préparation pharmaceutique, **caractérisée en ce qu'**en plus d'agents auxiliaires pharmaceutiques connus, elle contient un sel selon les revendications 1 ou 2.

18. Préparation pharmaceutique selon la revendication 17, pour application entérale, par exemple application orale ou rectale, ou pour application parentérale, par exemple intraveineuse, intramusculaire, sous-cutanée ou topique.

19. Utilisation du sel selon la revendication 1 ou 2 pour la préparation de formes de préparation galéniques.

20. Sel selon la revendication 1 ou 2, pour le traitement thérapeutique de l'organisme humain ou animal.

21. Sel selon la revendication 1 ou 2, pour le traitement thérapeutique de douleurs.

22. Préparation pharmaceutique selon la revendication 17 ou 18, pour le traitement thérapeutique de l'organisme humain ou animal.

23. Préparation pharmaceutique selon la revendication 17 ou 18, pour le traitement thérapeutique de la douleur.

24. Procédé de préparation d'un agent de traitement thérapeutique de l'organisme humain ou animal, **caractérisé en ce que** l'on utilise un sel selon l'un des revendications 1 ou 2 ou une préparation pharmaceutique selon l'une des revendications 17 ou 18.

25. Procédé de préparation d'un agent de traitement de la douleur, **caractérisé en ce que** l'on utilise un sel selon l'une des revendications 1 ou 2 ou une préparation pharmaceutique selon l'une des revendications 17 ou 18.
